Europäisches Patentamt

European Patent Office

Office européen des brevets

(11) Publication number: **0 312 662**
**A1**

## (12) EUROPEAN PATENT APPLICATION

(21) Application number: **87309360.3**

(22) Date of filing: **22.10.87**

(51) Int. Cl.⁴: **A61M 35/00**

(43) Date of publication of application:
**26.04.89 Bulletin 89/17**

(84) Designated Contracting States:
**AT BE CH DE ES FR GB GR IT LI LU NL SE**

(71) Applicant: **Vlock, David George**
**12 5th Avenue**
**New York, NY 10011(US)**

(72) Inventor: **Vlock, David George**
**12 5th Avenue**
**New York, NY 10011(US)**

(74) Representative: **Lawrence, Peter Robin**
**Broughton et al**
**GILL JENNINGS & EVERY 53-64 Chancery**
**Lane**
**London WC2A 1HN(GB)**

(54) **Fluid dispenser.**

(57) A fluid dispenser comprises a base portion 13 that is or can be temporarily secured to a handle, spaced apart, flexible, outer members 14 extending from outer positions around the base portion and a flexible inner member 14a extending from an inner position. The outer members externally define an open pyramid having an apex 15 and internally define a capillary cradle 16 in which fluid can be retained. There is a capillary passage 25 leading from the cradle to the apex whereby liquid in the capillary cradle is discharged upon flexural contact of the apex with a body or other surface.

FIG. 7.

EP 0 312 662 A1

## Fluid Dispenser

The invention relates to a fluid dispensing device for placing a quantity of fluid upon a body surface in a precise manner.

In dental, medical and surgical applications, it is often required to place a medicine or medicament upon a body surface such as the gums, skin, epithelium, cheek, or tongue. Often only a small amount of fluid is needed, particularly when spplying caustics and astringents. These types of fluids require precise placement to the affected areas without spillage. Often exacting amounts are required in order to prevent dripping and run over into non-affected regions.

Hand-held dispensing tools that can be used to extract fluid from a fluid source such as a vial or bottle, and to place the fluid upon a body surface are illustrated in US 1,632,686 and 1,586,302.

In both the above patents, a hand-held tool is described featuring a resilient tubular tip for carrying and dispensing fluids. While these hand-held devices accomplish placing small amounts of fluid upon various body surfaces, they cannot precisely extract a given quantity of fluid from a bottle or vial. The delivered fluid is a little more or a little less than what may be required. Delivery of the fluid is not carefully controlled. The amount that is delivered is greatly affected by the viscosity of the liquid.

In U.S. 4,583,982 there is described an improved fluid dispenser that is designed to extract a precise amount of fluid from a container and then accurately deliver that fluid exactly to the chosen body surface, and that is useful with fluids having different viscosities. This dispenser comprises a base portion that is or can be permanently or temporarily secured to a handle portion and at least three spaced apart, flexible, elongated outer members that extend from outer portions distributed around the base portion. The distal ends of the elongated members externally define the apex of an open pyramid and internally define a capillary cradle in which a fluid can be retained. A capillary passage leads from the cradle to the apex and liquid in the capillary cradle can be discharged upon flexural contact of the apex with the surface on to which the liquid is to be discharged.

Although this dispenser constitutes a great improvement on previous, hand-held, fluid dispensers, there is still a need to improve its reliability for delivering a wide range of liquids in accurately predetermined amounts.

In the invention, a flexible elongated inner member extends from an inner position on the base portion into the capillary cradle.

The invention includes both the base portion alone, provided with means for fitting it to an appropriate handle portion, and also combinations of the base portion with the handle portion. In such combinations, the handle and base portions may be a unitary structure or there may be means for fitting the base portion to the handle portion. When there are means for fitting the base portion to the handle portion these are preferably such as to permit releasable fitting, so that the base portion can be attached to the handle and, when desired, detached from the handle. The fitting means may be such that the base portion can be keyed to the handle portion and/or may comprise a male/female connection.

In use, it is essential that the distal ends of the elongated members should combine to define the stated apex and cradle but it is not essential that the base portion should initially be manufactured with this arrangement, provided it is manufactured in such a way that the distal ends of the elongated members adopt the desired configuration when the base portion is attached to the handle portion. Thus the act of attaching the base portion to the handle portion must be such as to cause the elongated members to adopt the desired configuration.

The base portion can be a web that interconnects the elongated members and that is connected to means by which it can be attached to the handle portion, but often the base portion is the surface of a truncated base that also includes, generally on its opposite surface, the means for connection to a handle.

There are at least three spaced apart, flexible, elongated outer members that have the form of prongs or tines and that define an open or skeleton pyramid. The distal ends, or tips, of the elongated members externally define the apex of the pyramid and internally define a capillary cradle. This cradle or pocket holds a precise, predetermined, amount of fluid as a result of the interaction of the surface tension of a chosen fluid and the inner surfaces of the outer members. The amount will depend upon the apex angle, the number of prongs and the partiuclar liquid, especially its surface tension. When the open pyramid is dipped into the liquid this cradle will automatically collect a predetermined amount of the liquid.

A capillary passage leads from the cradle to the apex and terminates at the extreme tip of the prongs. When this tip is contacted with a body or other surface and the prongs are slightly flexed, fluid will flow from the cradle through the capillary passage on to the surface. For best results the distal ends are not attached to each other and the capillary passage is formed between the adjacent facing surfaces of the distal ends.

The elongated outer members extend from outer positions distributed around the base portion so as to define an open pyramid having the desired base size and apex angle. Naturally the members are usually distributed substantially uniformly around the base and generally there are three of the outer members.

Additionally, in the invention, there is a flexible elongated inner member that extends from an inner position on the base portion into the capillary cradle. The provision of this inner member improves the ability of the device to hold, and subsequently release, a predetermined volume of fluid. In particular it enhances the ability of the dispenser to hold the fluid for substantial periods prior to discharge and it improves the ability of the device to be used effectively and accurately with a wide variety of fluids. The inner member promotes the distribution of capillarity in all lateral directions so that liquid can emerge upon any and all contacting surfaces in the vertical or any other direction. Also the inner member provides an extra surface that is close to all the other surfaces in the cradle and so acts as a common capillary interface within the cradle, thereby promoting the collection, retention and subsequent distribution of liquid from within the cradle.

The inner member must therefore extend into the cradle, for any particular liquid, sufficiently far that it can exert the desired effect on the liquid in the cradle. Also it must be internal of the pyramid, as otherwise it will merely form another external surface of the cradle. Generally there is a single said inner member extending from a position on the base portion that is substantially central.

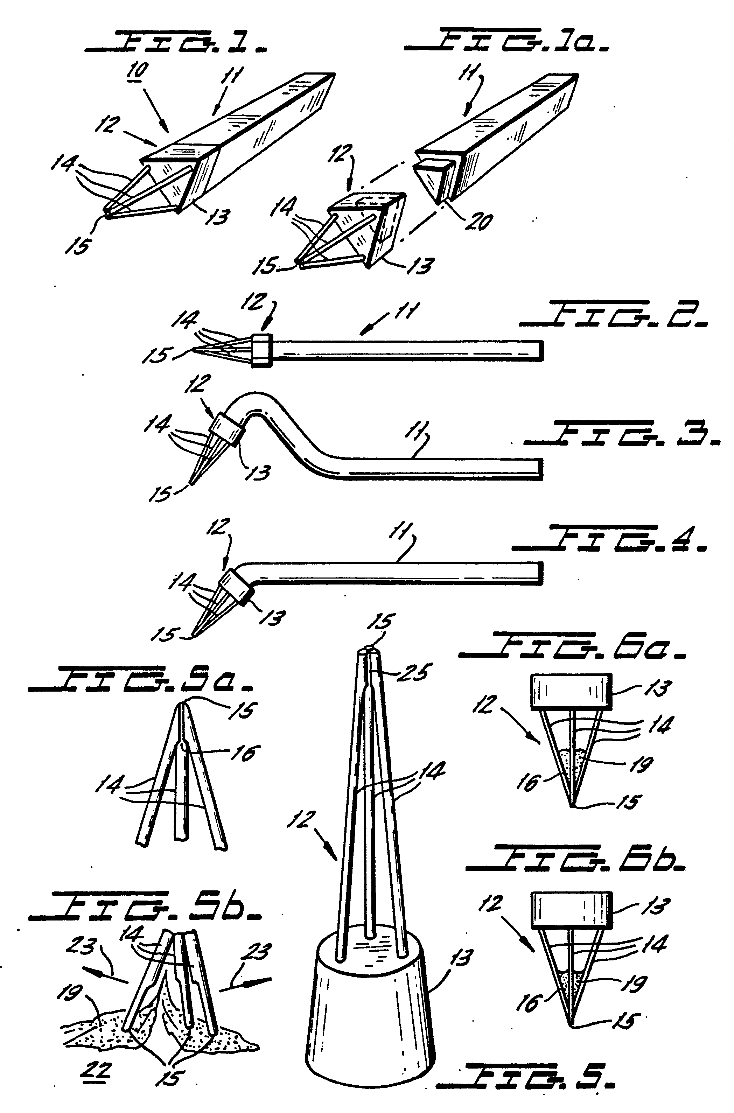

The invention is described in the accompanying drawings in which Figures 1 to 6 show the device of U.S. 4,583,982 and in particular illustrate various arrangements of base portions, outer members and handle portions that can be incorporated in the invention, and Figures 7 to 10 illustrate dispensers according to the invention. In particular,

Figure 1 is a perspective view of the fluid dispensing device of U.S. 4,583,982;

Figure 1a is a perspective exploded view of the device shown in Figure 1;

Figures 2 through 4 illustrate other embodiments of the device illustrated in Figure 1;

Figure 5 depicts an enlarged perspective view of the forward section of the device shown in Figure 1;

Figure 5a illustrates an enlarged view of the apex of the forward section shown in Figure 5;

Figure 5b shows an enlarged, exaggerated view of the apex of the forward section depicted in Figure 5a in a fluid discharging mode;

Figures 6a and 6b depict schematic views of the apex of the forward section shown in Figure 5 ina fluid supporting mode. Figure 6a shows the fluid with a concave menescus and

Figure 6b depicts the fluid with a convex meniscus;

Figure 7 is a perspective view of a fluid dispensing device according to the invention;

Figure 8a is an enlarged view of the fluid carrying and discharging member for use with the dispensing device;

Figure 8b is a perspective view of an illustrative dispensing device utilising he fluid carrying member shown in Figure 8a;

Figure 9a is an enlarged view of another fluid carrying and discharging member for use with the dispensing device;

Figure 9b is a perspective view of an illustrative dispensing device utilising the fluid carrying member shown in Figure 9a;

Figure 10 is a perspective exploded view of another dispensing device according to the invention.

Referring to Figure 1, the fluid dispenser 10 comprises a handle portion 11 and a fluid carrying and discharging section 12 that includes a base portion 13 on to which a number of spaced apart, flexible, outer members, prongs or tines 14 are affixed. The prongs 14 are arranged in the form of a pyramid skeleton. At least three prongs must be present in order to fulfill the unique fluid carrying capacity of the invention but the device can function with a multiplicity of prongs.

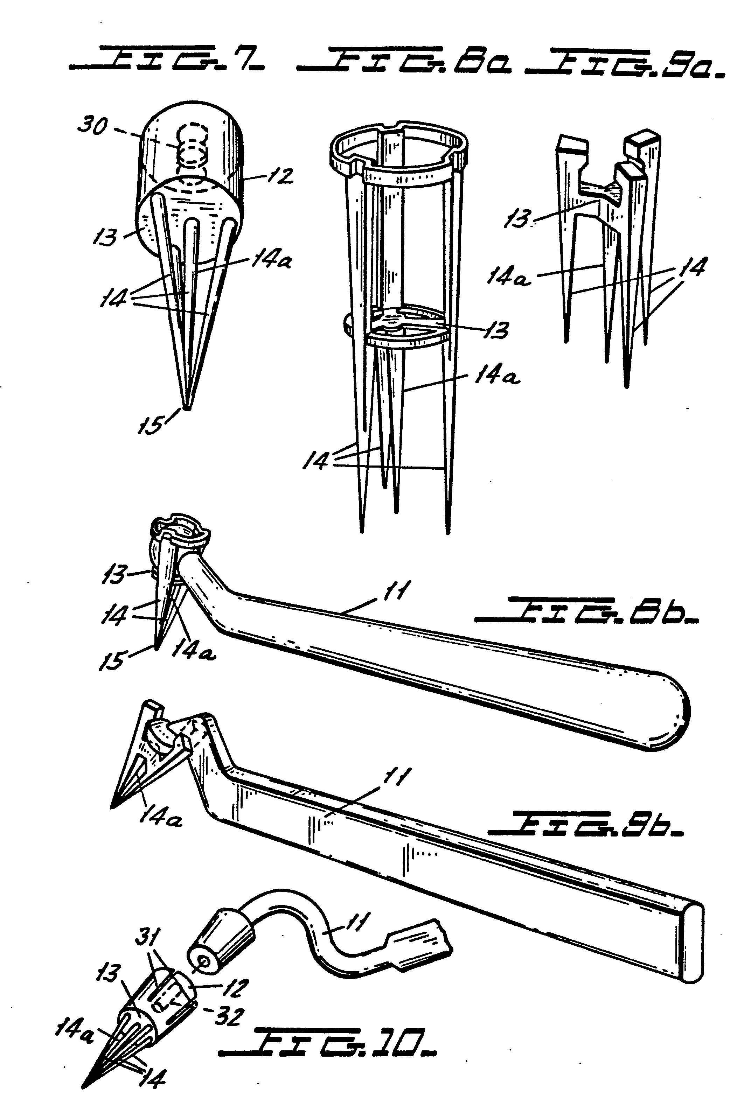

The distal ends of the prongs 14 form an apex 15 to the pyramid skeleton. As shown in more detail in Figures 5 and 5a each prong 14 is closely adjacent but not attached to its neighbouring prongs at the apex 15. The inner surfaces of the prongs combine to form a fluid carrying cradle 16. The tips of the prongs 14 are in close proximity to or slightly touch each other, but are not joined, and the inner surfaces of the neighbouring prongs together define a narrow capillary channel 25 extending from the cradle 16 to the apex 15.

When the apex 15 of the prongs 14 is immersed and removed from a fluid, a small, precise quantity of fluid 19 will be trapped in cradle 16, as is shown schematically in Figure 6a. The fluid quantity 19 is trapped in cradle 16 by means of the surface tension between the fluid 19 and the prongs 14. In most cases, the fluid quantity 19 will form a concave meniscus between the prongs 14, as shown in Figure 6a. However, with the use of hydrophobic materials for prongs 14, like Teflon, the meniscus may actually be convex, as depicted in Figure 6b.

Depending upon the number of prongs 14 used in the fluid section 12 and the types of materials and angles chosen for prongs 14, the quantity of fluid 19 can be varied to provide a precise amount of fluid extracted and carried in cradle 16.

The fluid quantity 19 can be precisely placed on a body surface, such as the skin or epithelium by touching the apex 15 of the prongs 14 to the body surface 22, as shown in the exaggerated enlarged view of Figure 5b. The fluid quantity 19 will be drawn from the cradle 16 down the capillary channel 25 to flood the desired surface 22 by flexing the prongs 14 (arrows 23) to disrupt the surface tension forces holding the fluid 19 in cradle 16. The fluid will flow by capillary action in any desired direction, including antigravitationally.

As shown in Figures 7 to 10, an elongated inner member, prong or tine 14a is positioned approximately centrally on base 13 and extends substantially into the capillary cradle defined by the outer members. Preferably there is a sharp acute angle between the centre prong 14a and each outside prong 14. For instance the angle may be about 8° as measured between the centre long axis of each prong.

The shape of the handle 11 and the angle of section 12 can be varied as shown in the alternative embodiments of Figures 2 to 4, 8b, 9b and 10. Thus the shape can be round as shown in Figures 2 and 3 and 8b or square or triangular as illustrated in Figures 1, 4 and 9b. Different handle shapes may be more conveniently held in accordance with the preference of the user.

The mating angle between section 12 and handle 11 may be varied for placing fluid on surfaces that are not easily accessible. Figures 3, 8b, 9b and 10 illustrate curved or non-rectilinear handles 11 with angled section 12 while Figure 4 shows an angled section 12 with a straight handle 11.

In keeping with the design or shape of the handle 11, the base 13 of section 12 can be a truncated cone or a square or rectangular prism.

The section 12 can be detachable from the handle 11 so that different sections 12 can be provided for different quantities of fluids or different fluids. Also sanitary and asceptic conditions will generally require a different section 12 for each patient, and so a section 12 can be discarded after use. It can be attached to the handle 11 by means of a male/female connection such as a triangular key 20 and mating triangular hole 21 as shown in Figure 1a or holes 30 as shown in Figure 7. The discharging sections 12 shown in Figures 8a and 9a have a base portion 13 in the form of a web that interconnects the outer members and are so constructed that when snapped on to the ends of the handles 11, as shown in Figures 8b and 9b respectively, the resulting pressure causes the members to adopt the desired configuration having the apex 15.

In Figure 10 the sides of the member 12 have slits 31 and a post 32 for insertion into handle 11 and to exert pressure on the prongs 14 that maintain the apex 15.

The prongs or tines 14 and 14a can be made of plastics or other inert materials that are flexible, sturdy, and chemically resistant. A multiplicity of prongs or tines can be utilised with one or more located approximately at the centre of the base member.

The handle and base portions of the device 10 can also be made from plastics that are strong and sturdy. It should be further understood that a unitary dispenser is also contemplated where the handle, the fluid carrying and discharging member, including the multiple prongs or tines, are fabricated or moulded from the same or compatible materials. This type of dispenser will generally be disarded after each use of the same purpose or for the same patient.

Where it is desired to sterilise the instrument, such plastics must be temperature resistant. Other materials such as titanium will provide inertness, strength and temperature and chemical resistance.

The dispenser of the invention is of particular value for taking a predetermined volume of fluid from a fluid source such as a vial or bottle and dispensing it accurately on to a body surface, and is of particular value when the fluid is a fluid medicament. However the invention is of value in any situation where it is desired to dispense a small and accurately predetermined volume of fluid on to a surface. Because the retention of the fluid is not dependent upon gravity the dispenser could possibly be used in outer space applications.

## Claims

1. A fluid dispenser suitable for placing liquid on a surface and which comprises a base portion (13) that is or can be permanently or temporarily secured to a handle portion (11) and at least three spaced apart, flexible, elongated outer members (14) that extend from outer positions distributed around the base portion and in which, when the base portion is attached to the handle portion, the distal ends of the elongated members externally define the apex (15) of an open pyramid and internally define a capillary cradle (16) in which fluid can be retained and there is a capillary passage (25) leading from the cradle to the apex, whereby liquid in the capillary cradle is discharged upon the flexural contact of the apex with the surface, characterised in that a flexible elongated inner member (14a) extends from an inner position on the base portion into the capillary cradle.

2. A dispenser according to claim 1 in which there is a single inner member extending from a substantially central position on the base portion.

3. A dispenser according to claim 1 or claim 2 in which there are three of the said outer members distributed substantially uniformly around the base portion.

4. A dispenser according to any preceding claim in which the said distal ends are not attached to each other and the capillary passage is formed between adjacent facing surfaces of the distal ends.

5. A dispenser according to any preceding claim consisting of the said base portion, and in which the base portion includes means for detachably fitting the base portion to a handle portion.

6. A dispenser according to any of claims 1 to 4 comprising the said base portion and the handle portion.

7. A dispenser according to claim 6 including means for detachably fitting the base portion to the handle portion.

8. A dispenser according to claim 6 in which the handle and base portions are a unitary structure.

FIG.1.

FIG.1a.

FIG.2.

FIG.3.

FIG.4.

FIG.5a.

FIG.6a.

FIG.6b.

FIG.5b.

FIG.5.

EP 0 312 662 A1

FIG.7  FIG.8a  FIG.9a.

FIG.8b.

FIG.9b.

FIG.10.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | US-A-4 583 982  (VLOCK)<br>* Figure 5 *<br>--- | 1 | A 61 M  35/00 |
| A | US-A-3 351 059  (KRAVITZ)<br>* Figure 5; column 4, lines 47-56 *<br>--- | 1 | |
| A,D | US-A-1 632 686  (WITHYCOMBE)<br>--- | | |
| A,D | US-A-1 586 302  (FUNK)<br>----- | 1 | |

TECHNICAL FIELDS
SEARCHED (Int. Cl.4)

A 61 M
B 43 K
A 61 B
B 01 L

The present search report has been drawn up for all claims

| Place of search | Date of completion of the search | Examiner |
|---|---|---|
| THE HAGUE | 30-06-1988 | RAKOWICZ,J.M. |

EPO FORM 1503 03.82 (P0401)